Europäisches Patentamt

European Patent Office

Office européen des brevets

(19)

(11) Publication number: **0 198 573**
**A1**

(12) # EUROPEAN PATENT APPLICATION

(21) Application number: **86300636.7**

(22) Date of filing: **30.01.86**

(51) Int. Cl.⁴: **A 61 B 5/02**

(30) Priority: **15.02.85 JP 28779/85**

(43) Date of publication of application: **22.10.86**
**Bulletin 86/43**

(84) Designated Contracting States: **AT BE CH DE FR GB IT LI NL SE**

(71) Applicant: **TSUYAMA MFG. CO., LTD.,**
**8-25 Kuwazu 2-chome Higashisumiyoshi-ku, Osaka-shi Osaka-fu (JP)**

(72) Inventor: **Tsuyama, Sadaharu, 4-21 Fuminosato 3-chome Abeno-ku, Osaka-shi Osaka-fu (JP)**

(74) Representative: **Seaborn, George Stephen et al, c/o Edward Evans & Co. Chancery House 53-64 Chancery Lane, London WC2A 1SD (GB)**

(54) **Pulse counter.**

(57) A light emitting portion (2) of a pulse counter is made to emit light rapidly winking with a frequency sufficiently higher than that of the pulse. The rapidly winking optical signal is received by a light receiving portion (3) of the pulse counter through the portion of the body subject to measurement. The light receiving portion (3) provides electric current according to the amount of the received light. Since the optical signal applied from the light emitting portion (2) is an intermittent optical signal in pulse form winking rapidly, the electric current provided according thereto is also intermittent current in pulse form. The light receiving portion (3) also provides electric current formed of a direct current component or a low frequency component according to the amount of the external light. A detecting means (5, 6) of the pulse counter removes the direct current component and the low frequency component obtained by the external light from the electric signal provided from the light receiving portion (3) to detect the pulse-form intermittent current obtained based on the optical signal from the light emitting portion (2).

ACTORUM AG

0198573

## PULSE COUNTER

BACKGROUND OF THE INVENTION

Field of the Invention

The present invention is utilized in a field in which the pulse of a human being is to be measured by a photoelectric conversion system. More particularly, the present invention relates to an improvement of a pulse counter in which a measuring method by a photoelectric conversion is adopted.

Description of the Prior Art

A blood current amount in the blood vessels of a human being changes cyclically according to the beating of the heart. Therefore, by detecting the change of the blood current amount, it is made possible to measure the pulse rate of a person undergoing inspection.

Such a cyclical change of the blood current amount can be detected externally by a photoelectric conversion system using a photosensor for example, without hurting the human body. Such detection by a photoelectric conversion system is based on the relation in which the light transmission coefficient of a human body changes according to the blood current amount in the fine capillary vessels of the human body. Thus, pulse counters using such a photoelectric conversion system have been proposed in the prior art.

In one of the widely known examples of such pulse counters, an earlobe or a fingertip of a person undergoing inspection is selected as a portion to be inspected. One reason for this is that it is easy to transmit light through such a thin portion and accordingly is also easy to detect the light transmission coefficient changing according to the change of the blood current amount, whereby the change of the blood current amount can be determined. Another reason is that such an end portion of a human body is hardly influenced by movement of the muscle and is of a thickness unchanged, causing little error in measurement. Consequently, such a pulse counter is designed to detect change of a transmitted light amount according to the change of the blood current amount of the inspected portion by holding this portion by means of a photosensor comprising a light emitting portion and a light receiving portion.

However, in such pulse counter, the light receiving portion receives not only the light from the light emitting portion but also the natural light or artificial light in the environment, applied directly thereto or transmitted through the inspected portion of the human body. If and amount of such external light applied to the light receiving portion of the photosensor is always constant, such external light will not particularly hinder correct measurement of the pulse. However, in reality, the amount of external light incident on the light receiving portion of the photosensor considerably changes if the inspected person with the photosensor attached to himself moves or changes his posture. Such change of the amount of external light substantially influences the change in the amount of the light from the light emitting portion to be detected by the light receiving portion, causing lack of accuracy in the detection of the change of light amount according to the pulse.

Thus, a pulse counter of such a conventional photoelectric conversion system makes unstable measurement under the influence of external light and particularly, in a light environment or in the state where the inspected person moves, an incorrect measurement cannot be avoided.

Therefore, it is necessary to exclude such influence of external light exerted upon a pulse counter. The U.S. Patent No. 4301808 discloses a pulse rate monitor using a photosensor operating in the infrared ray regions.

However, any of the above mentioned techniques cannot sufficiently exclude the influence of external light, which involves a problem that a correct measurement of the pulse cannot be made in a light environment under natural light or artificial light.

SUMMARY OF THE INVENTION

Therefore, the present invention intends to provide a pulse counter of a photoelectric conversion system improved for accurately measuring the pulse by excluding the influence of external light without intercepting the external light.

The present invention comprises:

a photosensor comprising a light emitting portion for providing an optical signal and a light receiving portion serving as a conversion element for converting the optical signal to an electric signal, the light receiving portion having linear characteristics of conversion from optical signal to electric signal, the light emitting portion and the light receiving portion being disposed close to each other and being respectively used in direct contact with a portion of a human body subjected to measurement,

light emitting portion driving means for applying a drive signal to the light emitting portion so that the light emitting portion emits light winking with a frequency higher than a prescribed frequency, and

detecting means coupled to the light receiving portion for detecting a desired signal component by removing a direct current component and a low frequency component from an electric signal provided by conversion in the light receiving portion.

At the time of detection of the pulse, the light emitting portion is made to emit light rapidly winking with a frequency higher than a prescribed frequency, for example with a frequency of approximately 1 kHz which is sufficiently higher than that of the pulse. The rapidly winking optical signal is received by the light receiving portion through the portion of the body subjected to measurement. For example, if the light emitting portion and the light receiving portion are separated and opposed to each other by the inspected portion such as an earlobe, the rapidly winking optical signal provided from the light emitting portion passes through the inspected portion with a certain ratio to attain the light receiving portion. If the light emitting portion and the light receiving portion are disposed adjacent to each other on the surface of the inspected portion, the rapidly winking optical signal provided from the light emitting portion is reflected in the inspected portion with a certain ratio to be received by the light receiving portion.

The light receiving portion provides electric current according to the amount of the received light. Since the optical signal applied from the light emitting portion is an intermittent optical signal in pulse form winking rapidly, the electric current provided according thereto is also intermittent current in pulse form.

On the other hand, since the light receiving portion receives not only the light from the light emitting portion but also external light from the environment, the light receiving portion also provides electric current according to the amount of the external light. However, since the amount of the external light changes little or changes very slowly, the electric current converted according to the external light in the light receiving portion becomes continuous current formed of a direct current component or a low frequency component. In consequence, the electric current provided from the light receiving portion is electric current comprising in an overlapping manner, the pulse-form intermittent current and the continuous current formed of a direct current component and a low frequency component.

The detecting means removes the direct current component and the low frequency component obtained by the external light from the electric signal provided from the light receiving portion to detect the pulse-form intermittent current obtained based on the optical signal from the light emitting portion.

Thus, based on the change in the envelope of the detected pulse-form output, the pulse rate is measured.

The present invention makes it possible to provide a pulse counter being capable of measuring accurately the pulse of a person subjected to inspection, without undergoing any influence of external light even under the most unfavorable conditions for measurement of the pulse in the photoelectric conversion system, namely, even under the conditions where the amount of external light applied to the light receiving portion of the sensor changes in a light environment.

In addition, it is not needed in the present invention to particularly intercept the application of external light to the light receiving portion of the sensor. Consequently, a structure for light interception from the environment can be omitted and thus, a pulse counter of simple structure, easily attached to a portion to be inspected can be provided.

These object and other objects, features, aspects and advantages of the present invention will become more apparent from the following detailed description of the present invention when taken in conjunction with the accompanying drawings.

BRIEF DESCRIPTION OF THE DRAWINGS

Fig. 1 is a diagram showing a concrete example of circuit structure of essential portions characterizing an embodiment of the present invention;

Fig. 2 is a schematic block diagram showing a total structure of an embodiment of the present invention;

Fig. 3 is a voltage waveform diagram for explaining the operation of the circuit shown in Fig. 1;

Fig. 4 is a graph showing photosensitivity characteristics of a photodiode used in an embodiment of the present invention; and

Fig. 5 is a graph showing photosensitivity characteristics of an ordinary phototransistor.

DESCRIPTION OF THE PREFERRED EMBODIMENTS

In the following, a preferred embodiment of this invention will be described with reference to the drawings.

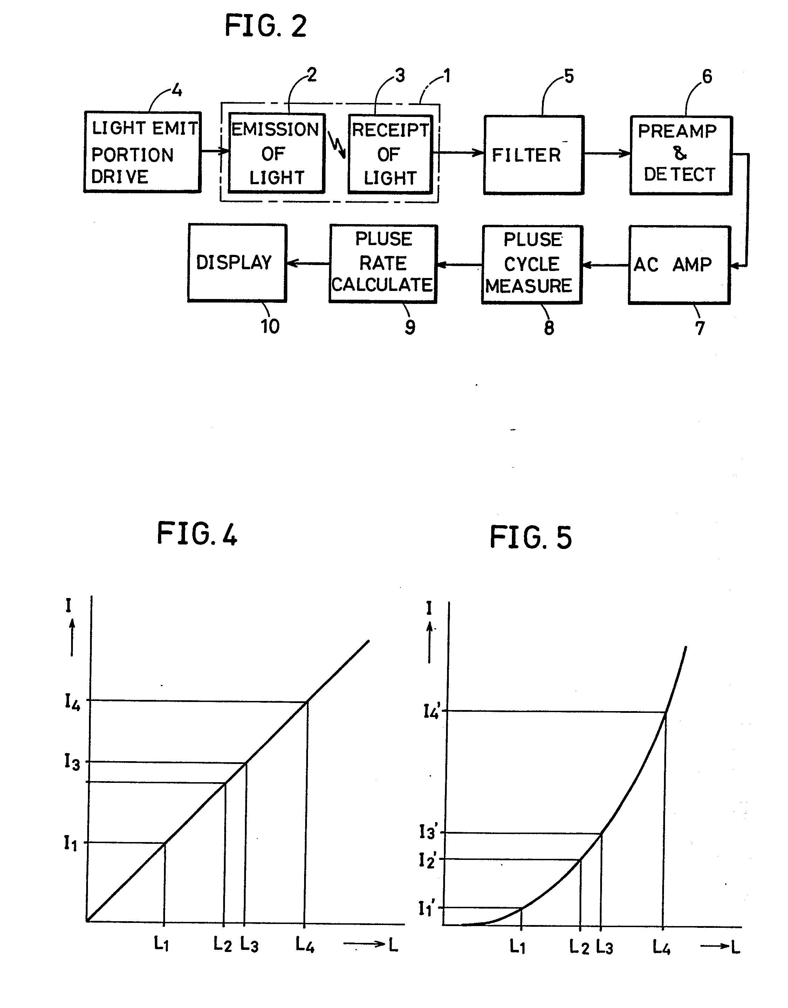

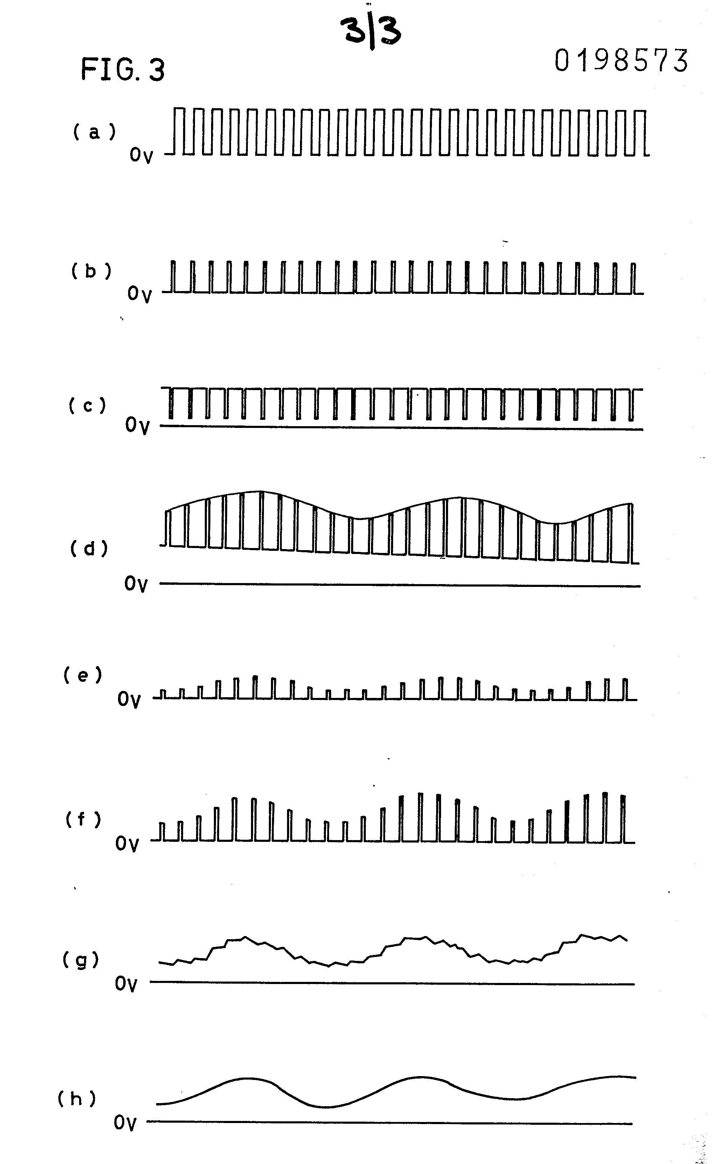

Fig. 2 is a schematic block diagram showing a structure of an embodiment of this invention. Referring to Fig. 2, a photosensor 1 comprises a light emitting element 2 and a light receiving element 3. In this embodiment, a light emitting diode is used as the light emitting element 2 and a photodiode is used as the light receiving element 3. The light emitting element 2 and the light receiving element 3 are disposed in prescribed positions close to each other. For example, in case where an earlobe serves as the portion to be inspected by the pulse counter of this embodiment, the light emitting element 2 and the light receiving element 3 are separated by a distance almost equal to the thickness of the earlobe so that the light emitting surface and the light receiving surface are opposed to each other. More preferably, the positions of the light emitting element 2 and the light receiving element 3 can be variably regulated in a mechanical manner so that they are in close contact with both surfaces of the earlobe if the thickness of the inspected portion, namely, the earlobe in this example changes dependent on individuals.

The light emitting element 2 is enabled by a light emitting portion drive circuit 4 containing an oscillation source to emit light rapidly winking with a frequency by far higher than that of the pulse, for example, with a frequency of approximately 1 kHz.

A output terminal of the light receiving element 3 receiving the optical signal provided from the light emitting element 2 is connected to a filter circuit 5 for cutting the direct current component and the low frequency component. The low frequency component cut by the filter circuit 5 is set for example nearly to a frequency of the rhythm formed by movement of a human body or a beating frequency of the pulse. More specifically, when the light emitting element 2 is made to emit rapidly winking light by the light emitting portion drive circuit 4, a rapid pulse-form signal of approximately 1kHz provided from the

light receiving element 3 in response thereto is taken out so that the unnecessary direct current component and low frequency component may be cut.

Then, the output of the filter circuit 5 is amplified and detected by the preamplifying and detecting circuit 6 so that change of the amplitude envelope of the pulse signal may be detected. Subsequently, the output is amplified by the AC amplifying circuit 7 and based on the amplified output, the pulse cycle is measured by the pulse cycle measuring circuit 8 and the pulse rate is calculated by the pulse rate calculating circuit 9 whereby the thus obtained result is displayed on the display 10.

Now, with regard to the structure of this embodiment shown in Fig. 2, a concrete example of structure comprising the light emitting portion drive circuit 4, the photosensor 1, the filter circuit 5 and the preamplifying and detecting circuit 6 which characterize this embodiment will be described in the following.

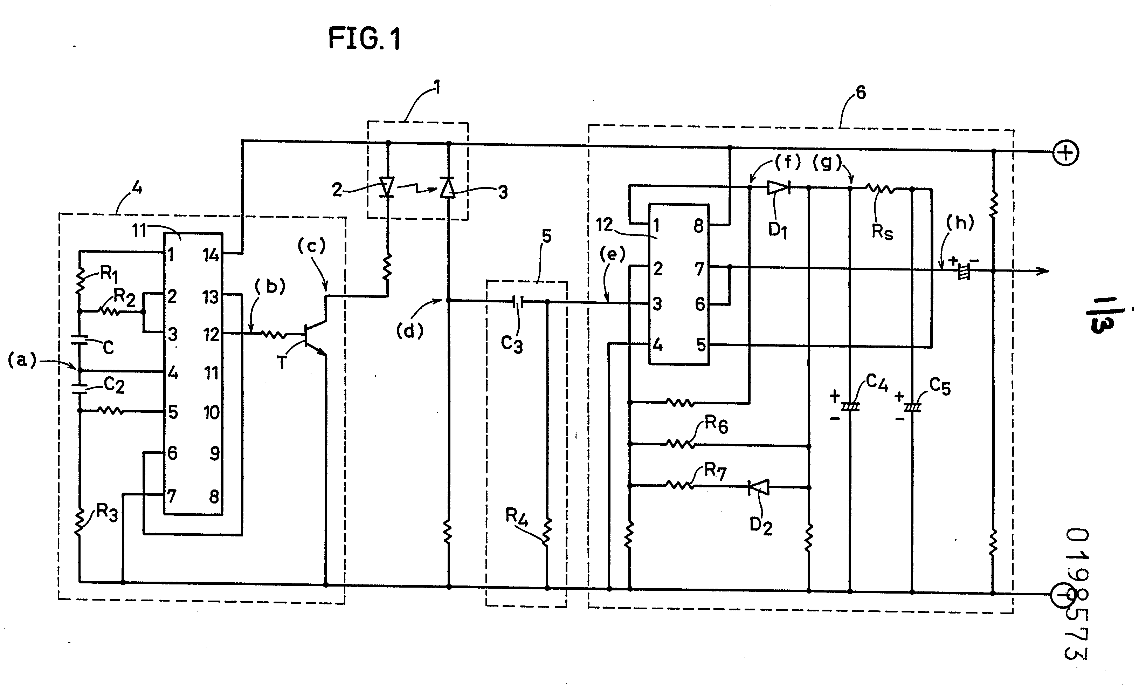

Fig. 1 is a circuit diagram showing a concrete example of structure of the portions characterizing this embodiment and Fig. 3 is a waveform diagram for explaining the operation of the circuit in Fig. 1. Referring to Figs. 1 and 3, the light emitting portion drive circuit 4 comprises an integrated circuit 11 as the center connected with resistors, capacitors, transistors etc. More specifically, the light emitting portion drive circuit 4 contains an oscillation source comprising resistors $R_1$ and $R_2$ and a capacitor $C_1$ between the pins (1) and (2) and between the pins (3) and (4) of the integrated circuit 11 respectively as well as a switching transistor T operating responsive to this oscillation source. The collector of the switching transistor T is connected with the light emitting diode 2 as the light emitting element.

Now, if the above stated oscillation source of the light emitting portion drive circuit 4 oscillates with a frequency of 1 kHz or more, output voltage having waveform as shown in Fig. 3(a) appears at the point (a) of the light emitting portion drive circuit 4. This output voltage is differentiated at the rise thereof in a differentiating circuit comprising a capacitor $C_2$ and a resistor $R_3$ and converted between the pins (5) and (6) and between the pins (13) and (12) of the integrated circuit 11 so that output voltage having waveform as shown in Fig. 3(b) is obtained to be applied to the base of the switching transistor T. The transistor T is turned on and off in response to the application of the output voltage (b), whereby the voltage at the collector terminal of the transistor T changes as shown in Fig. 3(c). Accordingly, the light emitting diode 2 emits light for each timing of descent of the voltage level shown in Fig. 3(c), in other words, each time the switching transistor T turns on to make electric current flow instantaneously, and thus, emission of light is repeated intermittently at high speed.

The pulse-form intermittent light emitting from the light emitting diode 2 is received by the photodiode 3 as the light receiving element. Now, assuming that a portion of the human body, not shown, is held between the light emitting diode 2 and the photodiode 3 and that the light emitting diode 2 emits rapidly winking light as described above, electric current according to the intermittent voltage waveform as shown in Fig. 3(d) flows at the point (d) on the cathode side of the photodiode 3. In this voltage waveform shown in Fig. 3(d), the change of voltage in pulse form represents change of electric current flowing in the photodiode 3 in response to the light from the light emitting diode 2. The amplitude of the pulse waveform changes with a certain ratio vased on the fact that the light transmission coefficient of the inspected portion of the human body changes due to the change in he amount of  blood current.

On the other hand, the lowest value of the voltage waveform is higher than OV because external light enters the photodiode 3 to cause some amount of continuous light to flow in the photodiode. In this figure, change of the amount of external light is represented as a straight line for the purpose of simplification. More specifically, Fig. 3(d) shows a case in which the amount of external decreases gradually.

Thus, electric current flowing as the output of the photodiode 3 is based on the sum of the amount of intermittent light applied from the light emitting diode 2 through the inspected portion to be properly detected and the amount of continuous light unnecessary for detection, applied from the environment.

On the other hand, the optical signal to electrical signal conversion characteristics (photosensitivity characteristics) of the photodiode 3 are linear characteristics as shown in Fig. 4. For example, assuming that the current flowing in the case of the external light amount $L_1$ is $I_1$ and that the current flowing in the case of the external light amount $L_3$ is $I_3$, let us compare the increase of current $(I_2 - I_1)$ obtained by addition of the amount of light applied from the light emitting diode 2 $(L_2 - L_1)$ in the case of the external light amount $L_1$ with the increase of current $(I_4 - I_3)$ obtained by addition of the amount of light applied from the light emitting diode 2 $(L_4 - L_3)$ in the case of the external light amount $L_3$. If the amounts of light applied from the light emitting diode 2 are equal, namely, $(L_2 - L_1) = (L_4 - L_3)$, the

increase of current is also equal, namely, $(I_2 - I_1) = (I_4 - I_3)$. In the other words, the photosensitivity characteristics of the photodiode 3 are constant if the total amount of incident light changes. Thus, in the circuit of this embodiment where the photosensor 1 is provided with the photodiode 3 as the light receiving element, even if external light amount changes in a light environment, only the direct current component of the detected output shown in Fig. 3(d) changes and the alternating current component, namely, the amplitude of the pulse waveform does not change.

The above described characteristics will be understood more clearly by comparison with the photosensitivity characteristics of a phototransistor. As is well known, a phototransistor generally has non linear photosensitivity characteristics as shown in Fig. 5 by the incident light amount L and the current I flowing in that case. For example, the increase of current $(I_2' - I_1')$ obtained by addition of the amount of light applied from the light emitting portion $(L_2 - L_1)$ in the case of the external light amount $L_1$ and the increase of current $(I_4' - I_3')$ obtained by addition of the amount of light applied from the light emitting portion $(L_4 - L_3)$ in the case of the external light amount $L_3$ are in the relation of $(I_2' - I_1') < (I_4' - I_3')$ even if in the case of $(L_2 - L_1) = (L_4$

- $L_3$). In other words, the photosensitivity of a phototransistor rises sharply according to the increase of the total of the incident light amount. As a result, if the photosensor 1 adopts as the light receiving device 3, such an element as a phototransistor having non linear photosensitivity characteristics, change of the amount of external light in a light environment exerts influence on the amplitude of the pulse waveform shown in Fig. 3(d), which makes it difficult to correctly detect change in the amplitude of the pulse signal.

Therefore, one of the features of this embodiment is that the photodiode 3 is used as the light receiving element as shown in Fig. 1.

The detected output from the photodiode 3 as shown in Fig. 3(d) is supplied to the high-pass filter circuit 5. The high-pass filter circuit 5 comprises a capacitor $C_3$ and a resistor $R_4$. Through this circuit 5, the low frequency component, namely, a portion containing the direct current component is removed from the detected output of the photodiode 3 so that output current according to the voltage waveform shown in Fig. 3(e) appears on the output side (e) of the circuit. This output current is intermittent current changing with a frequency of 1 kHz for example, having envelope multiplied by the change of the pulse as the alternating current component.

The output current as shown by the waveform (e) is amplified between the pins (3) and (1) of the integrated circuit 12 in the preamplifying and detecting circuit 6, so that amplified intermittent current shown in Fig. 3(f) is provided at the point (f). Then, only the envelope of the output current (f) is detected by diode $D_1$ and at the point (g), current based on the voltage waveform shown in Fig. 3(g) appears. Further, the voltage based on this voltage waveform (g) is stabilized by the resistor $R_5$ and the capacitor $C_5$ so as to be converted to alternating current having continuous waveform corresponding to the alternating current component. Then, the alternating current passes between the pins (5) and (7) of the integrated circuit 12 so that an output having voltage waveform as shown in Fig. 3(h) is provided from the point (h) to the AC amplifying circuit 7 (see Fig. 2) in the next stage.

In this embodiment, the preamplifying and detecting circuit 6 is made to have non linearity by the resistors $R_6$ and $R_7$ and the diode $D_2$ so as to prevent unfavorable influence exerted on the output at the above stated point (h) by the difference in the intensity of the signal from the photodiode 3 dependent on individuals.

It is to be added that in the respective voltage waveforms in Fig. 3, the cycles of intermittent voltage change are represented in an exaggerated manner for convenience sake to have dimension nearly 500 times as much as in reality, compared with the cycles of the alternating current component generated by the pulse.

Although the present invention has been described and illustrated in detail, it is clearly understood that the same is by way of illustration and example only and is not to be taken by way of limitation, the spirit and scope of the present invention being limited only by the terms of the appended claims.

0198573

CLAIMS

1. A pulse counter comprising:

a photosensor comprising a light emitting portion for providing an optical signal and a light receiving portion serving as a conversion element for converting said optical signal to an electric signal, said light receiving portion having linear characteristics of conversion from optical signal to electric signal, said light emitting portion and said light receiving portion being disposed close to each other and being respectively used in direct contact with a portion of a human body subjected to measurement,

light emitting portion driving means for applying a drive signal to said light emitting portion so that said light emitting portion emits light winking with a frequency higher than a prescribed frequency, and

detecting means coupled to said light receiving portion for detecting a desired signal component by removing a direct current component and a low frequency component from an output signal converted as an electric signal and provided from said light receiving portion.

2. A pulse counter in accordance with claim 1, wherein

said light receiving portion comprises a photodiode as the conversion element for converting an optical signal to an electric signal.

3. A pulse counter in accordance with claim 1, wherein

said frequency higher than the prescribed frequency is a frequency sufficiently higher than a frequency of rhythm formed by movement of a human body.

4. A pulse counter in accordance with claim 2, wherein

said frequency higher than the prescribed frequency is a frequency sufficiently higher than a frequency of rhythm formed by movement of a human body.

5. A pulse counter in accordance with claim 1, wherein

said frequency higher than the prescribed frequency is a frequency sufficiently higher than beating frequency of the pulse.

6. A pulse counter in accordance with claim 2, wherein

0198573

said frequency higher than the prescribed frequency is a frequency sufficiently higher than beating frequency of the pulse.

7. A pulse counter in accordance with claim 3, wherein

said frequency higher than the prescribed frequency is a frequency sufficiently higher than beating frequency of the pulse.

8. A pulse counter in accordance with claim 4, wherein

said frequency higher than the prescribed frequency is a frequency sufficiently higher than beating frequency of the pulse.

9. A pulse counter in accordance with claim 1, further comprising:

amplifying means coupled to said detecting means for amplifying a detected output provided from said detecting means,

cycle measuring means for measuring a cycle of said detected output amplified by said amplifying means, and

calculation and display means for calculating a pulse rate based on an output from said cycle measuring means to display the result of said calculation.

10. A pulse counter in accordance with claim 9, wherein

said amplifying means has an automatic gain control function.

FIG.1

## FIG. 2

## FIG. 4            FIG. 5

FIG. 3

3/3

0198573

## DOCUMENTS CONSIDERED TO BE RELEVANT

EP 86300636.7

| Category | Citation of document with indication, where appropriate, of relevant passages | Relevant to claim | CLASSIFICATION OF THE APPLICATION (Int. Cl.4) |
|---|---|---|---|
| D,A | US - A - 4 301 808 (TAUS)<br>* Abstract; column 6, lines 47-62; fig. 6,7 * | 1-9 | A 61 B 5/02 |
| A | US - A - 4 353 152 (O'CONNOR)<br>* Abstract; column 3, lines 36-64; fig. 2 * | 1-9 | |
| A | US - A - 4 258 719 (LEWYN)<br>* Abstract; column 6, lines 2-8,38-48; fig. 1 * | 1-9 | |
| A | DE - A - 2 136 823 (FRITZ HELLIGE)<br>* Page 3, lines 8-27; claims 1-5 * | 1-10 | |
| A | DE - B - 1 962 335 (HELLIGE)<br>* Claims 1,3,5; fig. * | 1,2,5-9 | TECHNICAL FIELDS SEARCHED (Int. Cl.4)<br><br>A 61 B |
| A | DE - A1 - 3 150 925 (HONEYWELL)<br>* Abstract; claims 1,2; page 4, lines 31-35 * | 1,2,9,10 | |
| A | DE - A1 - 3 006 477 (PHILIPS)<br>* Claims 1,3; page 6, lines 4-29 * | 1,2,9,10 | |

The present search report has been drawn up for all claims

| Place of search | Date of completion of the search | Examiner |
|---|---|---|
| VIENNA | 04-07-1986 | NEGWER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82